(19) Europäisches Patentamt
European Patent Office
Office européen des brevets

(11) Veröffentlichungsnummer: **0 096 279**
**B1**

# (12) EUROPÄISCHE PATENTSCHRIFT

(45) Veröffentlichungstag der Patentschrift: **06.11.85**

(51) Int. Cl.⁴: **C 07 D 489/08**, A 61 K 31/485

(21) Anmeldenummer: **83105077.8**

(22) Anmeldetag: **21.05.83**

## (54) N-(2-Methoxyethyl)-noroxymorphon, dessen Säureadditionssalze, diese enthaltende Arzneimittel und Verfahren zu deren Herstellung.

(30) Priorität: **03.06.82 DE 3220831**

(43) Veröffentlichungstag der Anmeldung: **21.12.83 Patentblatt 83/51**

(45) Bekanntmachung des Hinweises auf die Patenterteilung: **06.11.85 Patentblatt 85/45**

(84) Benannte Vertragsstaaten: **AT BE CH DE FR IT LI LU NL SE**

(56) Entgegenhaltungen:
**DE - A - 2 538 075**

**JOURNAL OF MEDICINAL CHEMISTRY, Band 18, Nr. 5, 1975 N. CHATTERJIE et al. "Stereospecific synthesis of the 6beta-hydroxy metabolites of naltrexone and naloxone", Seiten 490-492**
**JOURNAL OF MEDICINAL CHEMISTRY, Band 18, Nr. 3, 1975, E.F. HAHN et al. "Narcotic antagonists. 4. Carbon-6 derivatives of N-substituted noroxymorphones as narcotic antagonists", Seiten 259-262**
**N.E. WOLFF: "Burger's medicinal chemistry", 4. Auflage, Teil. 3, 1981, JOHN WILEY & SONS, New York, Seiten 711-712**

(73) Patentinhaber: **BOEHRINGER INGELHEIM KG, D-6507 Ingelheim am Rhein (DE)**

(72) Erfinder: **Merz, Herbert, Dr., Rotweinstrasse 53, D-6507 Ingelheim/Rhein (DE)**
Erfinder: **Langbein, Adolf, Dr., Am Goldberg 6, D-6535 Gau-Algesheim (DE)**
Erfinder: **Stockhaus, Klaus, Dr., Pfarrer-Heberer-Strasse 35, D-6530 Bingen/Rhein (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Gegenstand der Erfindung ist N-(2-Methoxyethyl)-noroxymorphon der Formel

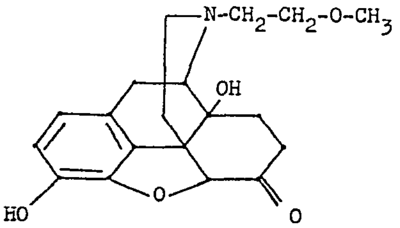

(I)

sowie die Säureadditionssalze dieser Verbindung, diese enthaltende Arzneimittel und Verfahren zu deren Herstellung.

Die Verbindung der Formel I ist nahe verwandt mit den bekannten Verbindungen der Formel

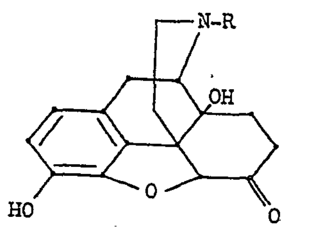

(II)

IIa: $-R = -CH_3$ (Oxymorphon[1])
IIb: $-R = -CH_2-CH=CH_2$ (Naloxon[2])
IIc: $-R = -CH_2-\triangle$ (Naltrexon[3])

1: US-PS 2806033
2: US-PS 3254088
3: US-PS 3332950

und den zu Vergleichszwecken hergestellten neuen Verbindungen der Formel

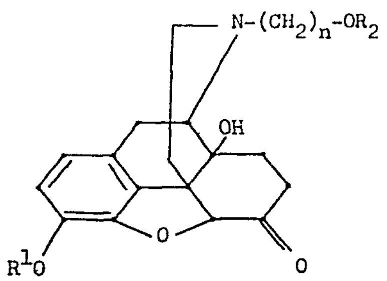

(III)

IIIa: $-R^1 = -H; -R^2 = -C_2H_5; n = 2$
IIIb: $-R^1 = -CH_3; -R^2 = -CH_3; n = 2$
IIIc: $-R^1 = -CH_3; -R^2 = -C_2H_5; n = 2$
IIId: $-R^1 = -H; -R^2 = CH_3; n = 3$

Die erfindungsgemässen Verbindungen der Formel I können nach folgenden zwei Verfahren hergestellt werden:

a) Alkylierung von Noroxymorphon der Formel

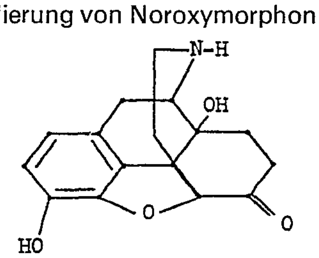

(IV)

mit 2-Methoxyethyl-halogenid der allgemeinen Formel

$$X-(CH_2)_2-OCH_3 \quad (V)$$

worin X ein Chlor-, Brom- oder Jodatom bedeutet.

Man verwendet zweckmässig die berechnete Menge oder einen geringen Überschuss des Alkylierungsmittels der allgemeinen Formel V und arbeitet zweckmässig in Gegenwart säurebindender Stoffe, wie z.B. Triethylamin, Dicyclohexylethylamin, Natriumcarbonat, Caliumcarbonat, Calciumoxid oder vorzugsweise Natriumhydrogencarbonat. Es ist vorteilhaft, die Reaktion in einem inerten Lösungsmittel durchzuführen, z.B. in Chloroform, Methylenchlorid, Benzol, Aceton, Dioxan, Tetrahydrofuran oder Dimethylformamid. Günstig sind auch Mischungen aus Dimethylformamid und Tetrahydrofuran. Die Reaktionstemperatur ist in weiten Grenzen variabel. Bevorzugt werden Temperaturen zwischen Raumtemperatur und dem Siedepunkt des verwendeten Lösungsmittels. Nach der Umsetzung werden die Reaktionsprodukte mit Hilfe bekannter Methoden isoliert, gereinigt und kristallisiert und gegebenenfalls in geeignete Säureadditionsverbindungen überführt.

b) Ketalspaltung einer Verbindung der Formel

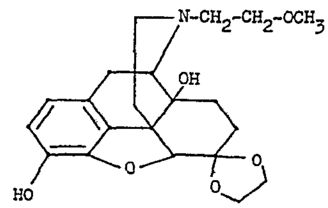

(VI)

durch Umsetzung mit verdünnten Säuren.

Das Noroxymorphon der Formel IV wird normalerweise aus Thebain in sterisch einheitlicher Form gewonnen. Die für das Verfahren b benötigte Ausgangsverbindung der Formel VI kann aus Noroxymorphon durch Ketalisierung mit Glykol in Gegenwart von Säure, Acylierung des Ketals der Formel VII mit Methoxy-essigsäurechlorid zum Acyl-Derivat der Formel VIII und anschliessende Reduktion mit Lithiumaluminiumhydrid nach folgendem Schema gewonnen werden:

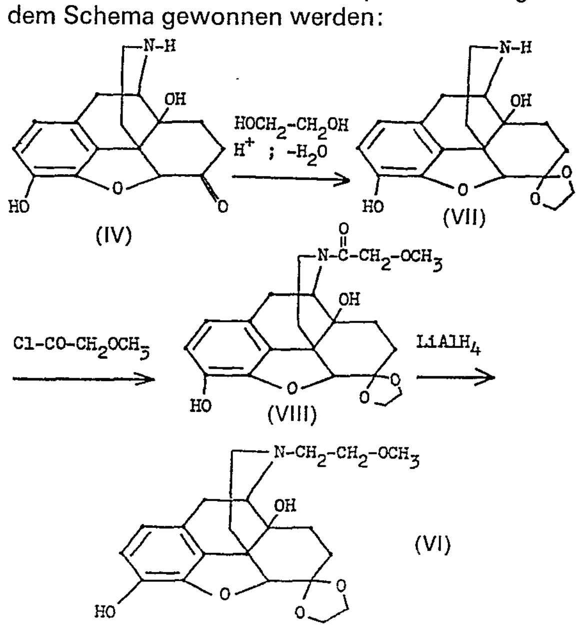


(IV) (VII) (VIII) (VI)

Die erfindungsgemässe Verbindung der Formel I ist eine Base und kann auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Valeriansäure, Pivalinsäure, Capronsäure, Oxalsäure, Malonsäure, Bernsteinsäure, Maleinsäure, Fumarsäure, Milchsäure, Weinsäure, Zitronensäure, Äpfelsäure, Benzoesäure, Phthalsäure, Zimtsäure, Salicylsäure, Ascorbinsäure, 8-Chlortheophyllin, Methansulfonsäure, Ethanphosphonsäure und dergleichen.

Die Verbindung der Formel I und deren Säureadditionssalze üben eine therapeutisch nutzbare Wirkung auf das Zentralnervensystem aus und können als nicht-suchtmachende Analgetika, d.h. Mittel zur Schmerzbekämpfung eingesetzt werden.

Die Verbindung der Formel I ist ein opioider Agonist-Antagonist mit einem nicht-morphinartigen, von anderen Substanzen nicht bekannten Wirkungsprofil. Einige für die Abgrenzung gegenüber verwandten Substanzen relevante pharmakologische Daten werden nachfolgend diskutiert.

Die starke analgetische Komponente lässt sich im Writhing-Test demonstrieren. In diesem Test ist die Verbindung der Formel I mit einer $ED_{50}$ von 0,013 mg/kg s.c. etwa 36mal stärker als Morphin, welches eine $ED_{50}$ von 0,47 mg/kg s.c. hat oder etwa 2,5mal stärker als das strukturell nahe verwandte Analgetikum Oxymorphon der Formel IIa ($ED_{50}$ = 0,032 mg/kg s.c.).

Das nicht-morphinartige Wirkungsprofil gibt sich zu erkennen in dem Fehlen typischer Nebenwirkungen der Opiate. Im Gegensatz zu den genannten Vergleichssubstanzen Morphin und Oxymorphon und anderen Opiaten zeigt das Hydrochlorid der Verbindung der Formel I z.B. weder das Straubsche Morphinschwanz-Phänomen, noch den sogenannten Manegetrieb. Der Unterschied zwischen dem Hydrochlorid der Verbindung I und den mit hohem Missbrauchspotential behafteten Opiaten lässt sich auch daran erkennen, dass die Substanz nicht in der Lage ist, bei morphinabhängigen Affen die nach Morphinentzug auftretenden Abstinenzerscheinungen aufzuheben. In diesem Modell benimmt sich das Hydrochlorid der Verbindung der Formel I vielmehr wie ein Antagonist, indem es die Abstinenzsymptome verschärft.

Die morphinantagonistische Komponente lässt sich im Haffner-Test durch die Aufhebung der von Morphin erzeugten Analgesie demonstrieren. Dabei hat die Verbindung der Formel I mit einer $AD_{50}$ von 0,3 mg/kg s.c. etwa 1/10 der antagonistischen Wirkungsstärke der strukturell nahe verwandten Vergleichssubstanz Naloxon der Formel IIb ($AD_{50}$ = 0,03 mg/kg s.c.). Bei Morphin-abhängigen Affen beobachtet man eine erhöhte Sensitivität gegenüber Morphin-Antagonisten, die dosisabhängig Abstinenzerscheinungen auslösen. In diesem Modell ist die Verbindung der Formel I so stark wie Naloxon. Im Gegensatz zur Verbindung der Formel I besitzen Naloxon und die zweite Substanz Naltrexon der Formel IIc keine analgetische Wirksamkeit, sondern sind sogenannte «reine Antagonisten».

Ein besonderer Vorzug der erfindungsgemässen Verbindung gegenüber anderen opioiden Analgetika, Agonisten und Agonist-Antagonisten ist eine ungewöhnlich hohe therapeutische Breite, die bei einer $LD_{50}$ von 1350 mg/kg s.c. bei der Maus bezogen auf die Wirkung im Writhing-Test bei 103 846 liegt. Die Vergleichswerte liegen für das Standardanalgetikum Morphin bei 1600, für den therapeutisch als Analgetikum genutzten Agonist-Antagonisten Pentazocin bei 169. Ein weiterer Vorteil der Verbindung der Formel I gegenüber neueren, noch nicht therapeutisch genutzten Substanzen aus der Klasse der opioiden Kappa-Agonisten der Benzomorphan-Reihe, denen ebenfalls eine hohe therapeutische Breite zugeschrieben wird (z.B. 18 000 für Ethylketazocin) ist das Fehlen einer starken Sedation. Diese ergibt sich bei den Vergleichssubstanzen als Lokomotionshemmung bei Mäusen im oder nahe beim therapeutischen Dosenbereich zu erkennen. Bei der erfindungsgemässen Verbindung wurde sie dagegen im untersuchten Bereich bis zu sehr hohen Dosen von 100 mg/kg nicht beobachtet.

Das eigenständige opioide Wirkungsprofil der erfindungsgemässen Verbindung der Formel I ergibt sich auch aus Studien an Organ-Modellen, wie dem Vas deferens der Maus und dem Meerschweinchen-Ileum und Rezeptor-Präparationen.

Systematische Abwandlung der Struktur der Formel I hat stets zu Substanzen mit wesentlich ungünstigeren Eigenschaften geführt. Die entsprechende N-(2-Ethoxyethyl)-Verbindung der Formel IIIa besitzt z.B. nur 1/20, die N-(3-Methoxypropyl)-Verbindung der Formel IIId hat nur 1/25 der Wirksamkeit der erfindungsgemässen Verbindung der Formel I. Ausserdem wirkt die Verbindung IIId morphinartig. Eine Veretherung der phenolischen Hydroxygruppe zu den Strukturen der Formeln IIIb und IIIc senkt die Wirksamkeit, für die Verbindung der Formel IIIb z.B. auf 1/84.

Die erfindungsgemässe Verbindung der Formel I sowie deren Säureadditionssalze können enteral oder auch parenteral angewandt werden. Die Dosierung für die enterale und parenterale Anwendung liegt bei etwa 0,5 bis 100 mg, vorzugsweise zwischen 1 und 20 mg. Die Verbindungen der Formel I bzw. deren Säureadditionssalze können auch mit anderen schmerzstillenden Mitteln oder mit andersartigen Wirkstoffen z.B. Sedativa, Tranquilizern, Hypnotika kombiniert werden. Geeignete galenische Darreichungsformen sind beispielsweise Tabletten, Kapseln, Zäpfchen, Lösungen, Suspensionen, Pulver oder Emulsionen; hierbei können zu deren Herstellung die üblicherweise verwendeten galenischen Hilfs- und Trägerstoffe oder Spreng- oder Schmiermittel oder Substanzen zur Erzielung einer Depotwirkung Verwendung finden. Die Herstellung derartiger galenischer Darreichungsformen erfolgt auf übliche Weise nach den bekannten Fertigungsmethoden.

Die Tabletten können aus mehreren Schichten

bestehen. Entsprechend können Dragées durch Überziehen von analog den Tabletten hergestellten Kernen mit üblicherweise in Dragéeüberzügen verwendeten Mitteln, beispielsweise Polyvinylpyrrolidon oder Schellack, Gummi arabicum, Talkum, Titandioxid oder Zucker hergestellt werden.

Zur Erzielung einer Depotwirkung oder zur Vermeidung von Unverträglichkeiten kann der Kern auch aus mehreren Schichten bestehen. Desgleichen kann auch die Dragéehülle zur Erzielung einer Depotwirkung aus mehreren Schichten aufgebaut sein, wobei die oben bei den Tabletten erwähnten Hilfsstoffe verwendet werden können.

Säfte der erfindungsgemässen Wirkstoffe bzw. Wirkstoffkombinationen können zusätzlich noch ein Süssungsmittel, wie Saccharin, Cyclamat, Glyzerin oder Zucker sowie ein geschmacksverbesserndes Mittel, z.B. Aromastoffe, wie Vanillin oder Orangenextrakt enthalten. Sie können ausserdem Suspendierungshilfsstoffe oder Dickungsmittel, wie Natriumcarboxymethylcellulose, Netzmittel beispielsweise Kondensationsprodukte von Fettalkoholen mit Ethylenoxid oder Schutzstoffe wie p-Hydroxybenzoate enthalten.

Injektionslösungen werden in üblicher Weise z.B. unter Zusatz von Konservierungsmitteln, wie p-Hydroxybenzoaten oder Stabilisierungsmitteln wie Komplexonen hergestellt und in Injektionsflaschen oder Ampullen abgefüllt.

Die Wirkstoffe bzw. Wirkstoffkombinationen enthaltende Kapseln können beispielsweise hergestellt werden, indem man die Wirkstoffe mit inerten Trägern, wie Milchzucker oder Sorbit mischt und Gelatinekapseln einkapselt.

Geeignete Zäpfchen lassen sich beispielsweise durch Vermischen der dafür vorgesehenen Wirkstoffe bzw. Wirkstoffkombinationen mit üblichen Trägermitteln wie Neutralfetten oder Polyethylenglykol bzw. dessen Derivaten herstellen.

Die folgenden Beispiele erläutern die Erfindung in nicht beschränkender Weise.

*Herstellungsbeispiele*

*Beispiel 1*

N-(2-Methoxyethyl)-noroxymorphon (Verfahren a)

3,24 g (0,01 Mol) Noroxymorphon-hydrochlorid, 2,6 g (0,03 Mol) Natriumbicarbonat und 1,52 g (0,011 Mol) 2-Methoxyethylbromid werden in 35 ml Dimethylformamid 24 Stunden bei 60° C kräftig gerührt. Anschliessend wird im Vakuum eingedampft und der Rückstand mit 45 ml Methylenchlorid, 5 ml Isopropanol und 20 ml Wasser geschüttelt. Die organische Phase wird 3mal mit je 20 ml Wasser gewaschen, mit Natriumsulfat getrocknet und im Vakuum eingedampft. Das Rohprodukt (3,3 g) wird durch Chromatographie an Kieselgel unter Verwendung von Methanol-Chloroform-konz. Ammoniak 90:10:0,5 als Fliessmittel gereinigt. Die Fraktionen mit der reinen Substanz werden eingedampft. Der Rückstand (2,7 g) kristallisiert bei der Behandlung mit 20 ml Diethylether. Die Kristallsuspension wird über Nacht im Kühlschrank aufbewahrt, dann abgesaugt, mit etwas Ether gewaschen und bei 70° C getrocknet. Man erhält dabei 1,7 g (49,3%) N-(Methoxyethyl)-noroxymorphon mit einem Schmelzpunkt von 164-168° C. Nach Umkristallisieren aus Methanol-Wasser 2:1 schmilzt die Substanz bei 170-173° C.

*Beispiel 2*

N-(2-Methoxyethyl)-noroxymorphon (Verfahren b)

a) N-Methoxyacetyl-noroxymorphon-ethylenketal

Zu einer kräftig gerührten Lösung/Suspension von 66,2 g (0,2 Mol) Noroxymorphon-ethylenketal in 662 ml absolutem Methylenchlorid und 80 ml Triethylamin wird unter Eiskühlung bei einer Reaktionstemperatur von 10° C innerhalb von ca. 2 Stunden eine Lösung von 47,8 g (0,44 Mol) Methoxyacetylchlorid in 331 ml absolutem Methylenchlorid eingetropft. Dabei entsteht eine klare Lösung, die 1 Stunde unter Rückfluss gekocht wird. Nach anschliessendem Abkühlen wird die Methylenchlorid-Lösung 3mal mit Wasser gewaschen, mit Natriumsulfat getrocknet und im Rotationsverdampfer, zuletzt im Vakuum, eingedampft. Der Rückstand wird mit 100 ml Toluol aufgenommen, und die Lösung wieder eingedampft. Der kristalline Rückstand (91,2 g) besteht aus N-Methoxyacetyl-noroxymorphon-ethylenketal, das ohne Zwischenreinigung weiterumgesetzt wird.

b) N-(2-Methoxyethyl)-noroxymorphon-ethylenketal

Der oben beschriebene Eindampfungsrückstand wird unter Erwärmen in 1400 ml absolutem Tetrahydrofuran gelöst. Nach Abkühlen auf 25-30° C wird die Lösung innerhalb von 4 Stunden zu einer kräftig gerührten Suspension von 24,0 g (0,63 Mol) Lithiumaluminiumhydrid in 600 ml absolutem Tetrahydrofuran getropft. Durch Kühlung sorgt man dafür, dass die Reaktionstemperatur nicht über 30-35° C steigt. Anschliessend wird die Reaktionsmischung 2 Stunden unter Rückfluss gekocht. Dann wird abgekühlt und unter Kühlung bei 0-5° C und kräftigem Rühren tropfenweise mit 300 ml Wasser versetzt. Nach Zugabe von 2,6 Liter gesättigter Diamoniumtartrat-Lösung wird 2 Stunden gerührt. Darauf wird die (leichtere) Tetrahydrofuran-Lösung abgetrennt und im Vakuum eingedampft. Die wässerige Lösung wird zweimal mit je 400 ml Methylenchlorid extrahiert. Der Eindampfungsrückstand der Tetrahydrofuran-Phase wird mit den Methylenchlorid-Extrakten gelöst, die Lösung zweimal mit je 400 ml Wasser gewaschen, mit Natriumsulfat getrocknet und im Vakuum eingedampft. Der Rückstand (69 g) besteht aus dem Ethylenketal von N-(2-Methoxyethyl)-noroxymorphon, das ohne weitere Reinigung der folgenden Ketalspaltung unterworfen wird.

c) N-(2-Methoxyethyl)-noroxymorphon-hydrochlorid

Zur Ketalspaltung wird der obige Rückstand mit einer Mischung von 70 ml Wasser und 40 ml konz. HCl 1 Stunde unter Rückfluss gekocht. Die klare, braune Lösung wird abgekühlt und mit 800 ml Aceton versetzt. Dabei kristallisiert N-(2-Methoxyethyl)-noroxymorphon-hydrochlorid aus. Nach Stehen über Nacht im Kühlschrank wird abgesaugt, mit Aceton gewaschen und bei 60° C getrocknet. Man erhält dabei 74,0 g (70,7%) N-(2-Methoxyethyl)-noroxymorphon-hydrochlorid mit einem Schmelzpunkt von 205° C. Umkristallisieren aus 167 ml Wasser (Lösen in der Siedehitze) und 1,4 Liter Aceton (Zugabe nach Abkühlen der wässerigen Lösung auf 50° C) gibt 62,0 g reine Substanz mit einem Schmelzpunkt von 265-267° C. Die durch nochmaliges Umkristallisieren aus Methanol erhaltene analysenreine Substanz schmilzt bei 269-270° C.

*Formulierungsbeispiele*

*Beispiel A: Tabletten*

| | |
|---|---|
| Wirkstoff gemäss Erfindung | 20,0 mg |
| Milchzucker | 120,0 mg |
| Maisstärke | 50,0 mg |
| kolloidale Kieselsäure | 2,0 mg |
| lösliche Stärke | 5,0 mg |
| Magnesiumstearat | 3,0 mg |
| | 200,0 mg |

Herstellung:

Der Wirkstoff wird mit einem Teil der Hilfsstoffe gemischt und mit einer Lösung der löslichen Stärke in Wasser granuliert. Nach dem Trocknen des Granulats wird der Rest der Hilfsstoffe zugemischt und die Mischung zu Tabletten verpresst.

*Beispiel B: Dragées*

| | |
|---|---|
| Wirkstoff gemäss Erfindung | 15,0 mg |
| Milchzucker | 100,0 mg |
| Maisstärke | 95,0 mg |
| kolloidale Kieselsäure | 2,0 mg |
| lösliche Stärke | 5,0 mg |
| Magnesiumstearat | 3,0 mg |
| | 220,0 mg |

Herstellung:

Der Wirkstoff und die Hilfsstoffe werden wie in Beispiel A beschrieben, zu Tablettenkernen verpresst, die mit Zucker, Talkum und Gummi arabicum üblicherweise dragiert werden.

*Beispiel C: Suppositorien*

| | |
|---|---|
| Wirkstoff gemäss Erfindung | 10,0 mg |
| Milchzucker | 150,0 mg |
| Suppositorienmasse q.s. ad | 1,7 g |

Herstellung:

Der Wirkstoff und der Milchzucker werden miteinander vermischt und die Mischung in der geschmolzenen Suppositorienmasse gleichmässig suspendiert. Die Suspensionen werden in gekühlte Formen zu Suppositorien von 1,7 g Gewicht ausgegossen.

*Beispiel D: Ampullen*

| | |
|---|---|
| Wirkstoff gemäss Erfindung | 1,0 mg |
| Natriumchlorid | 10,0 mg |
| bidestilliertes Wasser q.s. ad | 1,0 ml |

Herstellung:

Der Wirkstoff und das Natriumchlorid werden in bidestilliertem Wasser gelöst und die Lösung in Ampullen steril abgefüllt.

*Beispiel E: Tropfen*

| | |
|---|---|
| Wirkstoff gemäss Erfindung | 0,70 g |
| p-Hydroxybenzoesäuremethylester | 0,07 g |
| p-Hydroxybenzoesäurepropylester | 0,03 g |
| entmineralisiertes Wasser q.s. ad | 100,00 ml |

Herstellung:

Der Wirkstoff und die Konservierungsmittel werden in demineralisiertem Wasser gelöst und die Lösung filtriert und in Flaschen zu je 100 ml abgefüllt.

**Patentansprüche**

1. N-(2-Methoxyethyl)-noroxymorphon der Formel

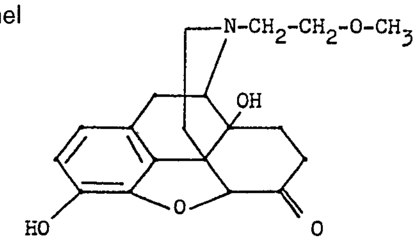

(I)

und dessen Säureadditionssalze.

2. Verfahren zur Herstellung von Verbindungen nach Anspruch 1, dadurch gekennzeichnet, dass man entweder

a) Noroxymorphon der Formel

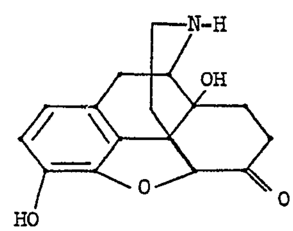

(IV)

mit einem 2-Methoxyethylhalogenid der allgemeinen Formel

$$X-(CH_2)_2-OCH_3 \qquad (V)$$

umsetzt; oder

b) N-(2-Methoxyethyl)-noroxymorphon-ethylenketal der Formel

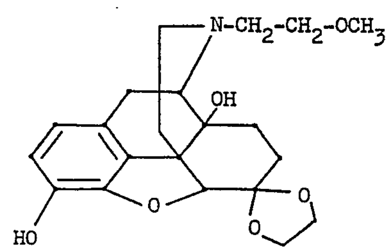

(VI)

durch Umsetzung mit verdünnten Säuren der Ketalspaltung unterwirft, und dass man gegebenenfalls die erhaltene Base der Formel I in ein Säureadditionssalz überführt.

3. Pharmazeutische Zubereitungen, dadurch gekennzeichnet, dass sie als Wirkstoff eine oder mehrere Verbindungen nach Anspruch 1 enthalten.

4. Verfahren zur Herstellung von pharmazeutischen Zubereitungen nach Anspruch 3, dadurch gekennzeichnet, dass man eine oder mehrere Verbindungen nach Anspruch 1 mit üblichen Hilfs- oder Trägerstoffen, Spreng- oder Schmiermitteln oder Substanzen zur Erzielung einer Depotwirkung zu pharmazeutischen Zubereitungen verarbeitet.

5. Verbindungen nach Anspruch 1 zur Verwendung für die Bekämpfung von Schmerzzuständen.

## Claims

1. N-(2-Methoxyethyl)-noroxymorphone of formula

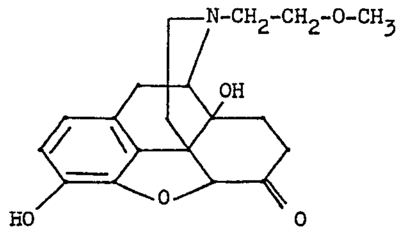

(I)

and the acid addition salts thereof.

2. Process for preparing compounds as claimed in claim 1, characterized in that either

a) noroxymorphone of formula

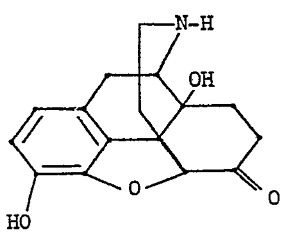

(IV)

is reacted with a 2-methoxyethyl halide of general formula

$$X-(CH_2)_2-OCH_3 \quad (V)$$

or

b) N-(2-methoxyethyl)-noroxymorphone-ethyleneketal of formula

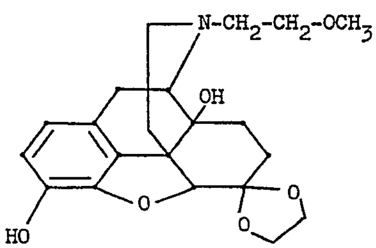

(VI)

is subjected to ketal splitting by reaction with dilute acids, and, if desired, the resulting base of formula I is converted into an acid addition salt thereof.

3. Pharmaceutical preparations, characterized in that they contain as active substance one or more compounds as claimed in claim 1.

4. Process for preparing pharmaceutical preparations as claimed in claim 3, characterized in that one or more compounds as claimed in claim 1 is or are processed with conventional excipients or carriers, disintegrants or lubricants or substances for obtaining delayed action in order to produce pharmaceutical preparations.

5. Compounds as claimed in claim 1 for use in combating pain.

## Revendications

1. N-(2-méthoxyéthyl)-noroxymorphone de formule:

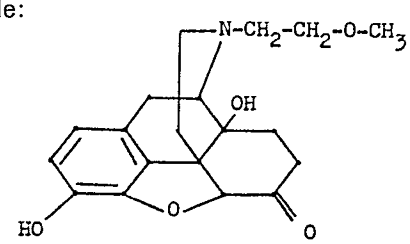

(I)

et ses sels d'addition d'acides.

2. Procédé pour la préparation de composés selon la revendication 1, caractérisé en ce que, soit:

a) on fait réagir la noroxymorphone de formule:

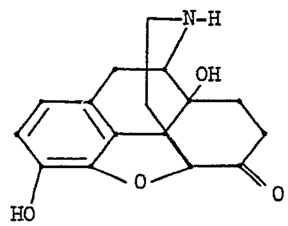

(IV)

avec un halogénure de 2-méthoxyéthyle de formule générale:

$$X-(CH_2)_2-OCH_3 \quad (V)$$

soit:

b) on soumet le cétal éthylénique de la N-(2-méthoxyéthyl)-noroxymorphone de formule:

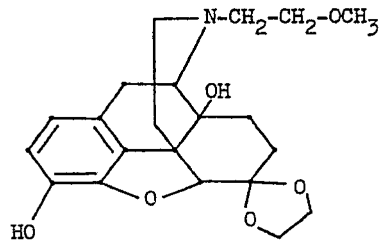

(VI)

à un clivage de cétal par réaction avec des acides dilués, et en ce qu'éventuellement on transforme la base obtenue de formule I en un sel d'addition d'acide.

3. Préparations pharmaceutiques, caractérisées en ce qu'elles contiennent en tant que substance active un ou plusieurs composés selon la revendication 1.

4. Procédé pour la préparation de préparations pharmaceutiques selon la revendication 3, caractérisé en ce qu'on transforme un ou plusieurs composés selon la revendication 1 au moyen d'adjuvants ou excipients, diluants ou lubrifiants ou substances pour obtenir un effet retard usuels en préparations pharmaceutiques.

5. Composés selon la revendication 1 pour l'utilisation pour lutter contre les états douloureux.